# EUROPEAN PATENT APPLICATION

(11) **EP 4 014 755 A1**
(43) Date of publication of application: **22.06.2022**
(21) Application number: 20214191.7
(22) Date of filing: 15.12.2020
(51) Int. Cl.: A23L 29/00, A23L 33/135, A23L 33/17, A23L 33/175, A23L 33/18, A23L 33/185, A61K 35/745, A61K 35/747

(54) **IMPROVING PROTEIN DIGESTION AND AMINO ACID BIOAVAILABILITY BY PROBIOTIC STRAINS**

(71) Applicant: DuPont Nutrition Biosciences ApS, 1411 Copenhagen K (DK)
(72) Inventor: MARTTINEN, Maija Emilia, 02460 Kantvik (FI); SAARINEN, Markku, 02460 Kantvik (FI); LAITILA, Arja, 02460 Kantvik (FI); NURMINEN, Päivi, 02460 Kantvik (FI); LEHTINEN, Markus, 02460 Kantvik (FI); ANJUM, Mehreen, 02460 Kantvik (FI)
(74) Representative: DuPont EMEA

(57) **Abstract**

This invention relates to the use of bacterial strains for improving protein digestion and/or increasing amino acid bioavailability in a subject, wherein said bacterial strains are of the species *Bifidobacterium animalis* subsp. *lactis, Lacticaseibacillus paracasei, Lactococcus lactis, Lactobacillus acidophilus, Lactiplantibacillus plantarum* and/or *Lacticaseibacillus rhamnosus* or a mixture thereof.

## Description

### FIELD OF THE INVENTION

This invention relates to the use of bacterial strains of the species *Bifidobacterium animalis* subsp. *lactis, Lacticaseibacillus paracasei, Lactococcus lactis, Lactobacillus acidophilus, Lactiplantibacillus plantarum* and/or *Lacticaseibacillus rhamnosus* or a mixture thereof for improving protein digestion and/or increasing amino acid bioavailability in a subject. This invention further relates to the use of bacterial strains as described in the present invention administered in the form of compositions, including food products, food ingredients, functional foods, dietary supplements, and pharmaceutically acceptable formulations.

### BACKGROUND

Proteins are essential in our daily diets for their nutritional value and role in food structure. Proteins are very active molecules that play multiple roles in the body. Some act as enzymes, meaning they act as biochemical catalysts by allowing the chemical reactions of the body to occur. Some proteins are hormones, others are hormone receivers (allow the cell to recognize a hormone), and others still are transporters (responsible for the transport of certain substances from the outside of the cell inward or vice versa).

Amino acids are the main components of proteins and although some amino acids can be produced by the body, some amino acids cannot - the so called essential amino acids -, and it is therefore necessary to ensure that our diet supply us with all the amino acids we need in order to synthesize the proteins needed for metabolism.

The nutritional quality of dietary protein depends on its amino acid composition and amino acid bioavailability. The digestibility and digestion rate of protein in the gastrointestinal tract impact protein bioavailability and amino acid absorption from the protein source (van der Wielen et al. 2017). The availability of dietary amino acids has been shown to be an important regulator of postprandial muscle protein metabolism (Koopman et al. 2009).

There is a growing market for sustainable and plant-based food choices. Digestibility of plant protein is, however, in general lower compared to animal and dairy protein (*i.e.* whey protein) (FAO 2012). This is due to lower protein solubility and antinutritional factors found in plant matrices (Gilani et al. 2012). The lower digestibility of plant protein results in lower bioavailability of amino acids, which may have an impact on the nutrition status, immune function, muscle mass and muscle strength of the individual (Wu 2016).

Protein needs is increased during childhood, in pregnancy and lactation in women and in the elderly (Wu 2016). In children, protein quality plays a role in supporting the growth (Ghosh 2016) and in elderly, protein quality is an essential factor in the prevention of sarcopenia (Baum et al. 2016). Some studies have shown that a rapid increase in amino acids in blood (aminoacidemia) after ingestion of a protein dose post-exercise improves adaptations to resistance training (Koopman et al. 2009; Jäger et al. 2017) and thus, provides benefits for athletes. Therefore, improving digestibility and digestion rate of plant proteins has important benefits on individual's nutrition and health status.

### OBJECT OF INVENTION

The object of the present invention is to improve protein digestibility and the bioavailability of amino acids from protein, in particular from plant protein, and more particularly from legume protein.

In particular, the object of the present invention is: (1) to improve the nutritive value of plant protein, (2) to increase plant protein digestion, (3) to improve plant protein bio-accessibility using probiotic strains, (4) to improve amino acid and peptide availability from plant protein, and/or (5) to improve plant protein utilization by humans and animals.

### SUMMARY OF THE INVENTION

The present invention is based on studies described herein which surprisingly demonstrate that strains of the species *Bifidobacterium animalis* subsp. *lactis, Lacticaseibacillus paracasei, Lactococcus lactis, Lactobacillus acidophilus, Lactiplantibacillus plantarum* and/or *Lacticaseibacillus rhamnosus* or a mixture thereof can improve protein digestion and increase amino acid bioavailability in a subject.

Accordingly, in one aspect, the present invention provides for the use of bacterial strains for improving protein digestion and/or increasing amino acid bioavailability in a subject, wherein said bacterial strains are of the species *Bifidobacterium animalis* subsp. *lactis, Lacticaseibacillus paracasei, Lactococcus lactis, Lactobacillus acidophilus, Lactiplantibacillus plantarum* and/or *Lacticaseibacillus rhamnosus* or a mixture thereof.

In another aspect according to the present invention, the protein is a plant protein.

In another aspect of the present invention, the plant protein is legume protein.

Plant protein can be soy protein, pea protein, fava bean protein, chickpea protein and/or lentil protein.

In a further aspect, the bacterial strains are strains B420, Bl-04, Lpc-37, LI-23, NCFM, Lp-115 and/or HN001 or a mixture thereof.

In yet a further aspect, the bacterial strains according to the present invention are administered in the form of compositions, such as food products, food ingredients, functional foods, dietary supplements, and pharmaceutically acceptable formulations.

### DESCRIPTION OF DRAWINGS

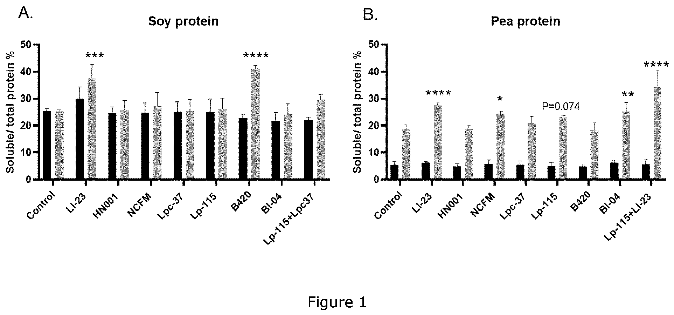

**Figure 1****.** Percentage of soluble protein per total protein in soy (A) and in pea protein (B) at baseline (black bars) and after simulated upper GI tract digestion (gray bars). The x-axis shows control and tested probiotic strains. No differences were detected at baseline values between the control and probiotic treatments. The values are average of three replicates. *P<0.05, **P<0.01, ***P<0.001, ****P<0.0001 statistically significant difference between probiotic treatment and control after digestion.

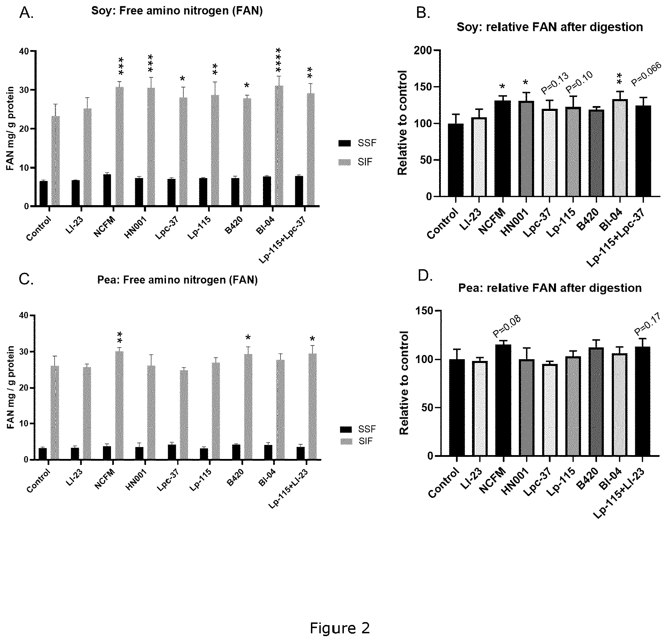

**Figure 2****.** Free amino nitrogen (FAN) content after in vitro digestion of soy protein (A-B) and pea protein (C-D). Figures A, C: Concentration of FAN (mg) per gram of protein in samples collected at baseline (SSF) and after intestinal phase (SIF) of the simulated upper GI tract digestion. Figures B, D: Relative FAN concentration compared to control after the simulated digestion. No differences were detected at baseline values between the control and probiotic treatments. The values are average of three replicates. *P<0.05, **P<0.01, ***P<0.001, ****P<0.0001 statistically significant difference between probiotic treatment and control. P>0.05 values presented in figures indicate a trend towards an increase.

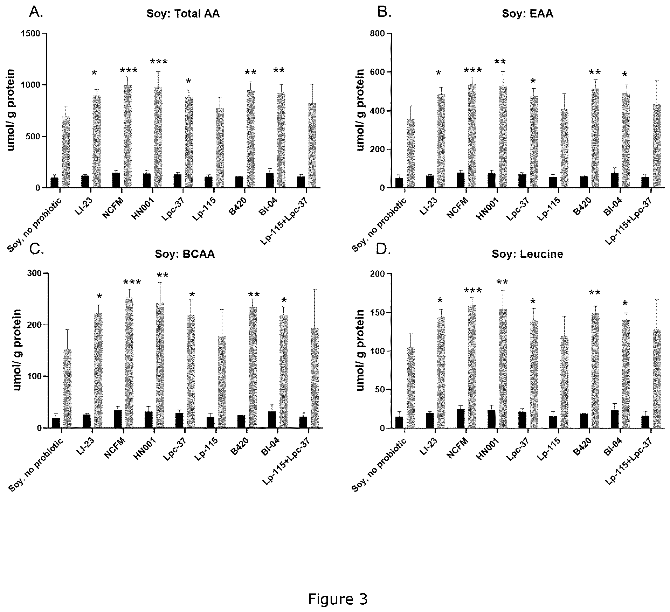

**Figure 3****.** Concentrations of total free amino acids (AA; A), essential amino acids (EAA; B), branched chain amino acids (BCAA; C) and free leucine (D) at baseline (SSF) and after in vitro digestion (SIF) of soy protein. No differences were detected at baseline values between the control (no probiotic) and probiotic treatments. *P<0.05, **P<0.01, ***P<0.001 statistically significant difference between probiotic treatment and control.

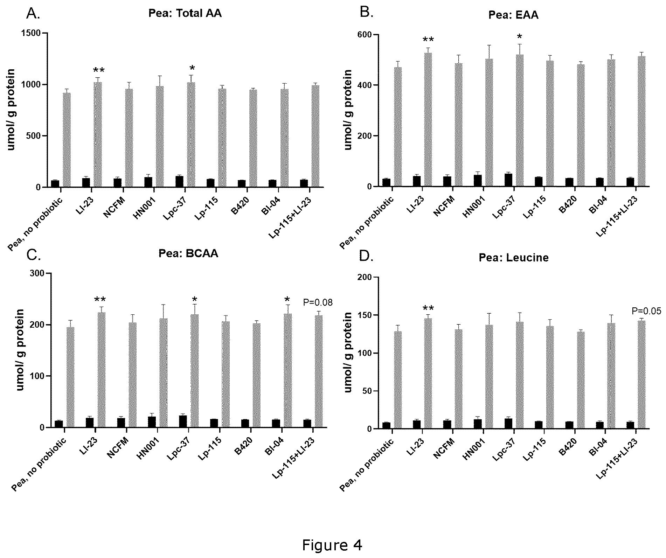

**Figure 4****.** Concentrations of total free amino acids (AA; A), essential amino acids (EAA; B), branched chain amino acids (BCAA; C) and free leucine (D) at baseline (SSF) and after in vitro digestion (SIF) of pea protein. No differences were detected at baseline values between the control (no probiotic) and probiotic treatments. *P<0.05, **P<0.01, **P<0.001 statistically significant difference between probiotic treatment and control. P>0.05 values presented in figures indicate a trend towards a higher concentration compared to control.

### DETAILED DESCRIPTION OF INVENTION

The results as described in the present invention show that bacterial strains improve protein digestibility and bioavailability of amino acids from protein, namely plant protein of legume origin. The effect of bacterial strains on plant protein digestion was investigated *in vitro* in conditions simulating digestion in the human upper gastrointestinal (GI) tract.

The detailed aspects of this invention are set out below. In part some of the detailed aspects are discussed in separate sections. This is for ease of reference and is in no way limiting. All of the embodiments described below are equally applicable to all aspects of the present invention unless the context specifically dictates otherwise.

### Bacteria

The bacterial strains used in aspects of the invention are bacterial strains of the species *Bifidobacterium animalis* subsp. *lactis, Lacticaseibacillus paracasei, Lactococcus lactis, Lactobacillus acidophilus, Lactiplantibacillus plantarum* and/or *Lacticaseibacillus* rhamnosus or a mixture thereof. In one particular aspect, the *Bifidobacterium animalis* subsp. *lactis* is strain B420 or Bl-04; in another aspect, *Lacticaseibacillus paracasei* is strain Lpc-37; in another aspect, *Lactococcus lactis* is strain LI-23; in another aspect, *Lactobacillus acidophilus* is strain NCFM; in another aspect, *Lactiplantibacillus plantarum* is strain Lp-115; in another aspect, *Lacticaseibacillus rhamnosus* is strain HN001. These strains are commercially available from DuPont Nutrition Biosciences ApS.

Preferably the bacterial strains used in the present invention are bacterial strains which are generally recognised as safe (GRAS) and, which are preferably GRAS approved. GRAS is an American Food and Drug Administration (FDA) designation that a chemical or substance added to food is considered safe by experts, and so is exempted from the usual Federal Food, Drug, and Cosmetic Act (FFDCA) food additive tolerance requirements.

Thus, in a first aspect, the present invention provides bacterial strains of the species *Bifidobacterium animalis* subsp. *lactis, Lacticaseibacillus paracasei, Lactococcus lactis, Lactobacillus acidophilus, Lactiplantibacillus plantarum* and/or *Lacticaseibacillus rhamnosus* or a mixture thereof for their use in improving protein digestion and/or increasing amino acid bioavailability in a subject.

Bacterial strains improved digestion rate of soy protein by 10-38 % and pea protein up to 15 % when compared to digestion without probiotic. Digestibility was improved by increasing protein solubility and/or increasing the concentration of protein digestion end-products that are more easily absorbed from the GI tract.

In another aspect, the present invention provides bacterial strains of the species *Bifidobacterium animalis* subsp. *lactis, Lacticaseibacillus paracasei, Lactococcus lactis, Lactobacillus acidophilus, Lactiplantibacillus plantarum* and/or *Lacticaseibacillus rhamnosus* or a mixture thereof for their use in improving plant protein digestion and/or increasing amino acid bioavailability in a subject.

In another aspect of the present invention, the plant protein is legume protein.

In another aspect, the plant protein according to the present invention is soy protein, pea protein, fava bean protein, chickpea protein and/or lentil protein.

In another aspect of the present invention, the protein is in powder form.

The bacterial strains, when used in aspects of the invention, are suitable for human and/or animal consumption. A skilled person will be readily aware of specific strains which are used in the food and/or agricultural industries and which are generally considered suitable for human and/or animal consumption.

Optionally, the bacterial strains when used in aspects of the invention are probiotic bacteria. The term "probiotic bacteria" is defined as covering any non-pathogenic bacteria which, when administered live in adequate amounts to a host, confers a health benefit on that host. For classification as a "probiotic", the bacteria must survive passage through the upper part of the digestive tract of the host. They are non-pathogenic, non-toxic and exercise their beneficial effect on health on the one hand via ecological interactions with the resident flora in the digestive tract, and on the other hand via their ability to influence the host physiology and immune system in a positive manner. Probiotic bacteria, when administered to a host in sufficient numbers, have the ability to progress through the intestine, maintaining viability, exerting their primary effects in the lumen and/or the wall of the host's gastrointestinal tract. They then transiently form part of the resident flora and this colonisation (or transient colonisation) allows the probiotic bacteria to exercise a beneficial effect, such as the repression of potentially pathogenic micro-organisms present in the flora and interactions with the host in the intestine including the immune system.

Thus, in a particular aspect of the present invention, the bacterial strains for use according to the invention are probiotic strains.

### Compositions

The term "composition" is used in the broad sense to mean the way something is composed, *i.e.* its general makeup. In aspects of the invention, the compositions may consist essentially of a single strain chosen from the species *Bifidobacterium animalis* subsp. *lactis, Lacticaseibacillus paracasei, Lactococcus lactis, Lactobacillus acidophilus, Lactiplantibacillus plantarum* and *Lacticaseibacillus rhamnosus.*

Alternatively, the compositions may comprise bacterial strains according to the present invention together with other components, such as biological and chemical components, active ingredients, metabolites, nutrients, fibres, prebiotics, etc.

In one aspect, the present invention provides for the use of bacterial strains for improving protein digestion and/or increasing amino acid bioavailability in a subject, wherein said bacterial strains are of the species *Bifidobacterium animalis* subsp. *lactis, Lacticaseibacillus paracasei, Lactococcus lactis, Lactobacillus acidophilus, Lactiplantibacillus plantarum* and/or *Lacticaseibacillus rhamnosus* or a mixture thereof and, and wherein said bacterial strains are administered in the form of compositions, such as food products, food ingredients, functional foods, dietary supplements, and pharmaceutically acceptable formulations.

In a particular aspect, the compositions according to the present invention further comprise prebiotics.

In yet a further aspect of the present invention, the bacterial strains according to the present invention are present in the composition in an amount between 10⁶ and 10¹², e.g. between 10⁸ and 10¹² colony forming units (CFU) per dose, optionally 10¹⁰ CFU per dose.

While it is not a requirement that the compositions comprise any support, diluent or excipient, such a support, diluent or excipient may be added and used in a manner which is familiar to those skilled in the art. Examples of suitable excipients include, but are not limited to, microcrystalline cellulose, rice maltodextrin, silicone dioxide, and magnesium stearate. The compositions of the invention may also comprise cryoprotectant components (for example, glucose, sucrose, lactose, trehalose, sodium ascorbate and/or other suitable cryoprotectants).

The terms "composition" and "formulation" may be used interchangeably.

Compositions used in aspects of the invention may take the form of solid, liquid, solution or suspension preparations. Examples of solid preparations include, but are not limited to: tablets, pills, capsules, granules and powders which may be wettable, spray-dried or freeze dried/lyophilized. The compositions may contain flavouring or colouring agents. The compositions may be formulated for immediate-, delayed-, modified-, sustained-, pulsed- or controlled-release applications.

By way of example, if the compositions of the present invention are used in a tablet form, the tablets may also contain one or more of: excipients such as microcrystalline cellulose, lactose, sodium citrate, calcium carbonate, dibasic calcium phosphate and glycine; disintegrants such as starch (preferably corn, potato or tapioca starch), sodium starch glycollate, croscarmellose sodium and certain complex silicates; granulation binders such as polyvinylpyrrolidone, hydroxypropylmethylcellulose (HPMC), hydroxypropylcellulose (HPC), sucrose, gelatin and acacia; lubricating agents such as magnesium stearate, stearic acid, glyceryl behenate and talc may be included.

Examples of other acceptable carriers for use in preparing compositions include, for example, water, salt solutions, alcohol, silicone, waxes, petroleum jelly, vegetable oils, polyethylene glycols, propylene glycol, liposomes, sugars, gelatine, lactose, amylose, magnesium stearate, talc, surfactants, silicic acid, viscous paraffin, perfume oil, fatty acid monoglycerides and diglycerides, hydroxymethylcelulose, polyvinylpyrrolidone, and the like.

For aqueous suspensions and/or elixirs, the composition of the present invention may be combined with various sweetening or flavouring agents, colouring matter or dyes, with emulsifying and/or suspending agents and with diluents such as water, propylene glycol and glycerin, and combinations thereof.

Specific non-limiting examples of compositions which can be used in aspects of the invention are set out below for illustrative purposes. These include, but are not limited to food products, food ingredients, functional foods, dietary supplements, pharmaceutical compositions and medicaments.

### Food products

The compositions of the invention may take the form of a food product. Here, the term "food" is used in a broad sense and covers food and drink for humans as well as food and drink for animals (i.e. a feed). Preferably, the food product is suitable for, and designed for, human consumption.

The food may be in the form of a liquid, solid or suspension, depending on the use and/or the mode of application and/or the mode of administration.

When in the form of a food product, the composition may comprise or be used in conjunction with one or more of: a nutritionally acceptable carrier, a nutritionally acceptable diluent, a nutritionally acceptable excipient, a nutritionally acceptable adjuvant, a nutritionally active ingredient.

By way of example, the compositions of the invention may take the form of one of the following:
A fruit juice; a beverage comprising whey protein: a health or herbal tea, a cocoa drink, a milk drink, a lactic acid bacteria drink, a yoghurt and/or a drinking yoghurt, a cheese, an ice cream, a water ice, a dessert, a confectionery, a biscuit, a cake, cake mix or cake filling, a snack food, a fruit filling, a cake or doughnut icing, an instant bakery filling cream, a filling for cookies, a ready-to-use bakery filling, a reduced calorie filling, an adult nutritional beverage, an acidified soy/juice beverage, a nutritional or health bar, a beverage powder, a calcium fortified soy milk, or a calcium fortified coffee beverage.

Optionally, where the product is a food product, the bacterium *Lacticaseibacillus paracasei* should remain effective through the normal "sell-by" or "expiration" date during which the food product is offered for sale by the retailer. Preferably, the effective time should extend past such dates until the end of the normal freshness period when food spoilage becomes apparent. The desired lengths of time and normal shelf life will vary from foodstuff to foodstuff and those of ordinary skill in the art will recognise that shelf-life times will vary upon the type of foodstuff, the size of the foodstuff, storage temperatures, processing conditions, packaging material and packaging equipment.

### Food ingredients

Compositions of the present invention may take the form of a food ingredient and/or feed ingredient.

As used herein the term "food ingredient" or "feed ingredient" includes a composition which is or can be added to functional foods or foodstuffs as a nutritional and/or health supplement for humans and animals.

The food ingredient may be in the form of a liquid, suspension or solid, depending on the use and/or the mode of application and/or the mode of administration.

### Functional Foods

Compositions of the invention may take the form of functional foods.

As used herein, the term "functional food" means food which is capable of providing not only a nutritional effect but is also capable of delivering a further beneficial effect to the consumer.

Accordingly, functional foods are ordinary foods that have components or ingredients (such as those described herein) incorporated into them that impart to the food a specific function - e.g. medical or physiological benefit - other than a purely nutritional effect.

Although there is no legal definition of a functional food, most of the parties with an interest in this area agree that they are foods marketed as having specific health effects beyond basic nutritional effects.

Some functional foods are nutraceuticals. Here, the term "nutraceutical" means a food which is capable of providing not only a nutritional effect and/or a taste satisfaction but is also capable of delivering a therapeutic (or other beneficial) effect to the consumer. Nutraceuticals cross the traditional dividing lines between foods and medicine.

### Dietary Supplements

The compositions of the invention may take the form of dietary supplements or may themselves be used in combination with dietary supplements, also referred to herein as food supplements.

The term "dietary supplement" as used herein refers to a product intended for ingestion that contains a "dietary ingredient" intended to add nutritional value or health benefits to (supplement) the diet. A "dietary ingredient" may include (but is not limited to) one, or any combination, of the following substances: bacteria, a probiotic (e.g. probiotic bacteria), a vitamin, a mineral, a herb or other botanical, an amino acid, a dietary substance for use by people to supplement the diet by increasing the total dietary intake, a concentrate, metabolite, constituent, or extract.

Dietary supplements may be found in many forms such as tablets, capsules, soft gels, gel caps, liquids, or powders. Some dietary supplements can help ensure an adequate dietary intake of essential nutrients; others may help reduce risk of disease.

### Pharmaceutical compositions (formulations)

Compositions of the invention may be used as - or in the preparation of -pharmaceuticals. Here, the term "pharmaceutical" is used in a broad sense - and covers pharmaceuticals for humans as well as pharmaceuticals for animals (*i.e.* veterinary applications). In a preferred aspect, the pharmaceutical is for human use.

The pharmaceutical can be for therapeutic purposes - which may be curative, palliative or preventative in nature.

A pharmaceutical may be in the form of a compressed tablet, tablet, capsule, ointment, suppository or drinkable solution.

When used as - or in the preparation of - a pharmaceutical, the compositions of the present invention may be used in conjunction with one or more of: a pharmaceutically acceptable carrier, a pharmaceutically acceptable diluent, a pharmaceutically acceptable excipient, a pharmaceutically acceptable adjuvant, a pharmaceutically active ingredient.

The pharmaceutical may be in the form of a liquid or as a solid - depending on the use and/or the mode of application and/or the mode of administration.

### Medicaments

Compositions of the invention may take the form of medicaments.

The term "medicament" as used herein encompasses medicaments for both human and animal usage in human and veterinary medicine. In addition, the term "medicament" as used herein means any substance which provides a therapeutic, preventative and/or beneficial effect. The term "medicament" as used herein is not necessarily limited to substances which need marketing approval but may include substances which can be used in cosmetics, nutraceuticals, food (including feeds and beverages for example), probiotic cultures, and natural remedies. In addition, the term "medicament" as used herein encompasses a product designed for incorporation in animal feed, for example livestock feed and/or pet food.

### Medical Foods

Compositions of the present invention may take the form of medical foods.

By "medical food" it is meant a food which is formulated to be consumed or administered with or without the supervision of a physician and which is intended for a specific dietary management or condition for which distinctive nutritional requirements, based on recognized scientific principles, are established by medical evaluation.

### Dosage

The compositions of the present invention may comprise from 10⁶ to 10¹² colony forming units (CFU) of bacterial strain(s) per dose or per gram of composition, and more particularly from 10⁸ to 10¹² CFU of bacterial strain(s) per dose or per gram of composition. Optionally the compositions comprise about 10¹⁰ CFU of bacterial strain(s) per dose or per gram of composition.

The bacterial strains(s) may be administered at a dosage from about 10⁶ to about 10¹² CFU of bacterial strain per dose, preferably about 10⁸ to about 10¹² CFU of bacterial strain per dose. By the term "per dose" it is meant that this number of bacteria is provided to a subject either per day or per intake, preferably per day. For example, if the bacteria are to be administered in a food product, for example in a yoghurt, then the yoghurt may contain from about 10⁶ to 10¹² CFU of the bacterial strain. Alternatively, however, this number of bacteria may be split into multiple administrations, each consisting of a smaller amount of microbial loading - so long as the overall amount of bacterial strain received by the subject in any specific time, for instance each 24 h period, is from about 10⁶ to about 10¹² CFU of bacteria, optionally 10⁸ to about 10¹² CFU of bacteria.

In accordance with the present invention an effective amount of at least one bacterial strain may be at least 10⁷ CFU of bacteria/dose, optionally from about 10⁸ to about 10¹² CFU of bacteria/dose, e.g., about 10¹⁰ CFU of bacteria/dose.

### Effects/Subiects/medical indication

In one embodiment, the term "subject", as used herein, means a mammal, including for example livestock (for example cattle, horses, pigs, and sheep) and humans, and also fish, such as salmon. In one embodiment the subject is a human individual with reduced food intake, an individual with reduced energy and/or protein and/or amino acid intake, an individual with high protein and/or energy demand/requirement, vegans, vegetarians, flexitarians, individuals with muscle loss, individuals with sarcopenia, individuals with muscle wasting, athletes and physically active individuals.

In another embodiment, the subject is a child, pregnant woman, lactating woman or an elderly individual.

In a further embodiment, the subject according to the present invention is an animal, such as livestock, pets and companion animals, racing animals, such as racehorses and race camels, in need of improving growth, increasing carcass mass and/or increasing muscle mass.

### Prebiotics

In one embodiment, the bacterial strains and compositions of the present invention may further be combined or comprise one or more fibres and/or prebiotics.

Prebiotics are defined as a substrate that is selectively utilized by host microorganisms conferring a health benefit. These are generally ingredients that beneficially affect the health of the host by selectively stimulating the growth and/or activity of one or a limited number of bacteria, and thus improve host health. The prebiotic can be applied to oral route, but it can be also applied to other microbially colonized sites. Typically, prebiotics are carbohydrates (such as oligosaccharides), but the definition does not preclude non-carbohydrates, such as polyphenols, or polyunsaturated fatty acids or other ingredients that can be utilized selectively by a limited number of bacteria to confer a health benefit. The most prevalent forms of prebiotics are nutritionally classed as soluble fibres. To some extent, many forms of dietary fibres exhibit some level of prebiotic effect.

Examples of suitable prebiotics include alginate, xanthan, pectin, locust bean gum (LBG), inulin, guar gum, galacto-oligosaccharide (GOS), fructo-oligosaccharide (FOS), polydextrose 10 (i.e. Litesse^{®}), lactitol, L-Arabinose, D-Xylose, L-Rhamnose, D-Mannose, L-Fucose, inositol, sorbitol, mannitol, xylitol, fructose, carrageenan, alginate, microcrystalline cellulose (MCC), betaine, lactosucrose, soybean oligosaccharides, isomaltulose (Palatinose TM), isomalto-oligosaccharides, gluco-oligosaccharides, xylooligosaccharides, manno-oligosaccharides, beta-glucans, cellobiose, raffinose, gentiobiose, melibiose, xylobiose, cyciodextrins, isomaltose, trehalose, stachyose, panose, pullulan, verbascose, galactomannans, (human) milk oligosaccharides and all forms of resistant starches.

### Method embodiments of the invention

For the avoidance of doubt, the bacterial strains and compositions for improving protein digestion and/or increasing amino acid bioavailability in a subject as described in the present invention can also be utilised in methods.

Therefore, in one aspect, the present invention provides a method of improving protein digestion and/or increasing amino acid bioavailability in a subject, wherein said bacterial strains are of the species *Bifidobacterium animalis* subsp. *lactis, Lacticaseibacillus paracasei, Lactococcus lactis, Lactobacillus acidophilus, Lactiplantibacillus plantarum* and/or *Lacticaseibacillus rhamnosus* or a mixture thereof.

### EXAMPLES

The following examples are provided to demonstrate and further illustrate specific embodiments and aspects of the present invention and are not to be construed as limiting the scope thereof.

### MATERIALS AND METHODS

### Protein digestion in a simulated upper GI tract model

Overnight cultures of selected probiotic strains were prepared in bacteria-specific growth media. Bacteria were grown until late logarithmic stage, and harvested by centrifugation (10 minutes, 4000 x g, 4 °C). The bacteria-containing pellet was washed three times and suspended in 0.9% NaCl. Optical density (OD600) was measured and bacteria number counted using OD600/concentration curve that was determined for each strain by flow cytometry in advance. The tested strains were the following commercial probiotics: *Bifidobacterium animalis ssp. lactis* B420, *Bifidobacterium animalis ssp. Lactis* Bl-04, *Lactobacillus acidophilus* NCFM, *Lacticaseibacillus rhamnosus* HN001, *Lacticaseibacillus paracasei Lpc-37, Lactiplantibacillus plantarum Lp-115, and Lactococcus lactis Ll-23.*

Protein powders used in simulations were: soy protein isolate (SUPRO^{®} XT 219D, DuPont; 86.7 % protein) and pea protein isolate (TRUPRO^{™} 2000, DuPont; 84.9 % protein). Gamma-irradiation of 12 kGy was performed to inactivate indigenous bacteria in the protein powders.

In vitro digestion of protein was performed according a static standardized method simulating the conditions in the healthy adult upper gastrointestinal tract (Minekus et al. 2014, Brodkorb et al. 2019). Briefly, the static digestion process was simulated in three stages: the oral stage where the protein powder (2 g in 20 mL) with probiotic treatment (2x10⁸ bacteria in 20 mL) or without bacteria were mixed with the simulated salivary fluid (SSF) and amylase enzyme (A3176, Sigma-Aldrich, Germany). pH was adjusted to 6.5 and the bottles were placed on a magnetic stirrer (200 rpm) in a water bath for 2 min at 37°C. Then simulated gastric fluid (SGF) was added to the bottles with pepsin enzyme (P7012, Sigma-Aldrich, Germany) and the pH was adjusted to 2.8 and incubated for 2 h at 37°C in a water bath with continuous magnetic stirring. Following the gastric stage simulated intestinal fluid (SIF) was added followed by pancreatin (P3292, Sigma-Aldrich, Germany) and bile solution (B8631, Sigma-Aldrich, Germany). The pH was adjusted to 6.8 and the incubation was continued for 2 h at 37°C in a water bath with mixing. All the probiotic treatments and controls were performed in three biological replicates.

Sampling was performed at the baseline from simulated salivary fluid (SSF samples) and at the end of digestion from simulated small intestinal fluid (SIF). The samples were centrifuged at 10,000 x g at 4 °C for 30 minutes, and supernatant was carefully separated, aliquoted and immediately frozen at -80 °C.

### Microbiological analyses

Plate counting method was used to determining bacteria survival in the digestive fluids after each phase. Each sample was plated on MRS plates after appropriate 10-fold serial dilution in peptone water and incubated at 37°C for 24 h for colony counting. Plates were incubated anoxically at 37°C in Mitsubishi Anaeropack jar (Thermo Scientific, U.S.A.) with two Anaerogen sachets (Oxoid, Thermo Scientific, Germany). Colonies were counted daily for three subsequent days and percentage survival was calculated by dividing the total number of colonies obtained after 3 days with the total number of bacteria that was inoculated at the baseline.

### Protein solubility assay

The BCA (bicinchoninic acid) assay (Thermo Scientific^{™} Pierce^{™} BCA protein assay, Rockford, IL) was used for measuring soluble protein content. The samples were diluted 100-fold. Absorbance was measured at OD562 nm in EnSight multimode plate reader (PerkinElmer). Bovine serum albumin, provided with the kit, was used as the protein standard. Each sample was analyzed in triplicate.

### Free amino nitrogen assay

Free amino nitrogen (FAN) was measured to evaluate the extent of proteolysis in a sample using the OPA (o-phthaldialdehyde) method. Samples were diluted 10-fold. FAN was quantified using manual assay procedure (K-PANOPA kit, Megazyme, Ireland) according to manufacturer's standard protocol. Absorbance was measured at 340 nm wavelength. Each sample was analyzed in duplicate.

### Analysis of free amino acids

Free amino acids in digestion samples were determined using an automated pre-column derivatization procedure with o-phthalaldehyde (OPA) and reversed-phase high performance liquid chromatography (HPLC) as described by Greene et al. (2009) with modifications. In short, samples were prepared by addition of norvaline as an internal standard after which amino acids were extracted and the proteins precipitated with 0.1% trifluoroacetic acid incubating the sample at 4oC for 2h. After centrifugation, an aliquot of the supernatant was transferred into a Ultrafree-mc 10000 NMWL microcentrifuge filter unit (Merck KGaA, Darmstadt, Germany) and centrifuged at 10000 x G for about 1h. The filtrate was used for the analysis. An Agilent 1260 Infinity II (Agilent, Waldbronn, Germany) chromatography system consisting of a quaternary pump, a column oven, a programmable injector and a diode array detector was used for derivatization, separation and detection of amino acids. Sample was derivatized in the injector needle with a mixture of OPA and 3-mercaptopropionic acid reagent (10 mg/ml each, Agilent 5061-3335) in 0.4 M borate buffer pH 10.2 (Agilent 5061-3339). The separation of OPA-amino acid derivatives was performed on an Agilent Zorbax Eclipse Plus C18 column (2.1 x 50 mm, 1.8 µm, 95Å) at 40oC. A buffer solution consisting of 10 mM sodium phosphate - 10 mM sodium borate at pH 8.2 was used as mobile phase A and a mixture of acetonitrile, methanol and water (45:45:10) as mobile phase B. A gradient elution of A and B at flow rate of 0.42 ml/min was employed for the separation: 0-0.2 min., A = 98% and B = 2%; 0.2-7.7 min., a linear decrease of A to 43% and a linear increase of B to 57%; 7.8-8.3 min., A = 0% and B = 100%; 8.4-9 min., A = 98% and B = 2%. The OPA derivatives were detected at 338 nm and internal standardization method was used for the quantitation.

### RESULTS

The results showed that the bacterial strains improve plant protein digestibility and availability of amino acids from plant protein. Bacterial strains improved digestion rate of soy protein by 10-38 % and pea protein up to 15 % compared to digestion without strains. Digestibility was improved by increasing protein solubility and/or increasing the concentration of protein digestion end-products that are more easily absorbed from GI tract.

### 1) Probiotic strains improve digestibility of plant protein by increasing protein solubility in the upper GI tract

Solubility of protein is associated with digestibility properties of protein. Soluble protein is more easily accessed by digestive enzymes in the gastrointestinal tract. Soluble protein content was measured at baseline and after simulated digestion.

Solubility of soy protein was improved by strains B420 and Ll-23 compared to digestion without probiotics (Figure 1A). Solubility of pea protein was improved by LI-23, combination of Lp-115 and LI-23, Bl-04 and NCFM (Figure 1B).

Overall, solubility of protein from legume origin was significantly improved by probiotic bacteria (Figure 1).

All probiotic bacteria survived the digestive conditions and were alive after the digestion.

### 2) Probiotic strains improve digestion of plant protein in the upper GI tract

The digestion rate of protein was evaluated measuring free amino nitrogen (FAN) concentration in samples at baseline and after simulated digestion. After digestion of plant protein, concentration of FAN increased in all probiotic strain treatments and control treatment confirming increased hydrolysis after simulated digestion in the upper GI tract.

Digestion of soy protein in the presence of a probiotic strains resulted in higher FAN concentration compared to control. The greatest increase was produced by strainsBl-04, HN001, and NCFM, followed by Lp-115, Lpc-37 and B420 (Figure 2A-B).

Digestion of pea protein resulted in higher FAN with NCFM, B420 and combination of Lp-115 and LL-23 when compared to control without probiotic (Figure 2C-D).

Overall, digestion rate of protein from legume origin was significantly improved by probiotic bacteria (Figure 2). Probiotics showed specificity in their action, certain bacteria being more effective with soy protein and some being more effective with pea protein.

### 3) Probiotics strains increase concentration of free amino acids after digestion of plant protein

Concentration of free amino acids was measured in samples at baseline and after simulated digestion. After in vitro digestion of plant protein, concentration of total free amino acids (AA), essential amino acids (EAA), and branched chain amino acids (BCAA) increased in all treatments. Treatment with NCFM, HN001, Bl-04, B420, Lpc-37 and Ll-23 resulted in increased levels of free AA, EAA, BCAA and leucine in soy protein compared to control (Figure 3). Treatment with LL-23 and Lpc-37 resulted in increased levels of free AA, EAA, BCAA and leucine in pea protein compared to control (Figure 4). Treatment with Bl-04 resulted in higher levels of free BCAA in pea protein compared to control (Figure 4C).

All publications mentioned in the above specification are herein incorporated by reference. Various modifications and variations of the described methods and system of the present invention will be apparent to those skilled in the art without departing from the scope and spirit of the present invention. Although the present invention has been described in connection with specific preferred embodiments, it should be understood that the invention as claimed should not be unduly limited to such specific embodiments. Indeed, various modifications of the described modes for carrying out the invention which are obvious to those skilled in biochemistry and biotechnology or related fields are intended to be within the scope of the following claims.

### REFERENCES CITED IN THE DESCRIPTION

van der Wielen N, Moughan PJ, Mensink M. Amino acid absorption in the large intestine of humans and porcine models. The Journal of nutrition. 2017; 147(8):1493-8.
Koopman R, Crombach N, Gijsen AP, Walrand S, Fauquant J, Kies AK, Lemosquet S, Saris WH, Boirie Y, van Loon LJ. Ingestion of a protein hydrolysate is accompanied by an accelerated in vivo digestion and absorption rate when compared with its intact protein. The American journal of clinical nutrition. 2009; 90(1): 106-15.
FAO 2012. Gilani S, Tome D, Moughan P, Burlingame B. Report of a sub-committee of the 2011 FAO consultation on "Protein quality evaluation in human nutrition" on: the assessment of amino acid digestibility in foods for humans and including a collation of published ileal amino acid digestibility data for. FAO Expert. 2012;58.
Gilani GS, Xiao CW, Cockell KA. Impact of antinutritional factors in food proteins on the digestibility of protein and the bioavailability of amino acids and on protein quality. British Journal of Nutrition. 2012;108(S2): S315-32.
Wu G. Dietary protein intake and human health. Food Funct. 2016;7(3): 1251-1265.
Ghosh S. Protein quality in the first thousand days of life. Food and nutrition bulletin. 2016;37(1_suppl):S14-21.
Baum JI, Kim IY, Wolfe RR. Protein consumption and the elderly: what is the optimal level of intake? Nutrients. 2016;8(6):359.
Jäger R, Kerksick CM, Campbell BI, Cribb PJ, Wells SD, Skwiat TM, Purpura M, Ziegenfuss TN, Ferrando AA, Arent SM, Smith-Ryan AE. International society of sports nutrition position stand: protein and exercise. Journal of the International Society of Sports Nutrition. 2017; 14(1):1-25.
Minekus M, Alminger M, Alvito P, Ballance S, Bohn TO, Bourlieu C, Carriere F, Boutrou R, Corredig M, Dupont D, Dufour C. A standardised static in vitro digestion method suitable for food-an international consensus. Food & function. 2014;5(6): 1113-24.
Brodkorb A, Egger L, Alminger M, Alvito P, Assunção R, Ballance S, Bohn T, Bourlieu-Lacanal C, Boutrou R, Carrière F, Clemente A. INFOGEST static in vitro simulation of gastrointestinal food digestion. Nature protocols. 2019;14(4):991-1014.
Greene J, Henderson JW Jr., Wikswo JP. (2009) Rapid and precise determination of cellular amino acid flux rates using HPLC with automated derivatization with absorbance detection. Agilent technologies, application note 5990-3283EN.

## Claims

1. Use of bacterial strains for improving protein digestion and/or increasing amino acid bioavailability in a subject, wherein said bacterial strains are of the species *Bifidobacterium animalis* subsp. *lactis, Lacticaseibacillus paracasei, Lactococcus lactis, Lactobacillus acidophilus, Lactiplantibacillus plantarum* and/or *Lacticaseibacillus rhamnosus* or a mixture thereof.

2. The use according to claim 1, wherein the protein is plant protein.

3. The use according to claim 2, wherein the plant protein is legume protein.

4. The use according to claim 2, wherein the plant protein is soy protein, pea protein, fava bean protein, chickpea protein and/or lentil protein.

5. The use according to any one of the preceding claims, wherein the protein is in powder form.

6. The use according to claim 1, wherein the bacterial strains are probiotic bacterial strains.

7. The use according to claim 1, wherein the bacterial strain of the species *Bifidobacterium animalis* subsp. *lactis* is strain B420 and/or strain Bl-04.

8. The use according to claim 1, wherein the bacterial strain of the species *Lacticaseibacillus paracasei* is strain Lpc-37.

9. The use according to claim 1, wherein the bacterial strain of the species *Lactococcus lactis* is strain LI-23.

10. The use according to claim 1, wherein the bacterial strain of the species *Lactobacillus acidophilus* is strain NCFM.

11. The use according to claim 1, wherein the bacterial strain of the species *Lactiplantibacillus plantarum* is strain Lp-115.

12. The use according to claim 1, wherein the bacterial strains of the species *Lacticaseibacillus rhamnosus* is strain HN001.

13. The use according to any one of the preceding claims, wherein said bacterial strains are administered in the form of compositions, such as food products, food ingredients, functional foods, dietary supplements, and pharmaceutically acceptable formulations.

14. The use according to claim 12, wherein said compositions further comprise prebiotics, such as fibres.

15. The use according to any one of the preceding claims, wherein said subject is a human individual with reduced food intake, an individual with reduced energy and/or protein and/or amino acid intake, an individual with high protein and/or energy demand/requirement, vegans, vegetarians, flexitarians, individuals with muscle loss, individuals with sarcopenia, individuals with muscle wasting, athletes and physically active individuals.

16. The use according to claim 15, wherein the individual is a child, pregnant woman, lactating woman or an elderly individual.

17. The use according to claims 1-14, wherein said subject is an animal, such as livestock, pets and companion animals, racing animals, such as racehorses and race camels, in need of improving growth, increasing carcass mass and/or increasing muscle mass.
